(19) Europäisches Patentamt / European Patent Office / Office européen des brevets

(11) **EP 3 001 833 B1**

(12) **EUROPEAN PATENT SPECIFICATION**

(45) Date of publication and mention of the grant of the patent:
**23.01.2019 Bulletin 2019/04**

(51) Int Cl.:
**B82Y 15/00** (2011.01)    **C07K 16/00** (2006.01)
**G01N 33/53** (2006.01)    **G01N 33/531** (2006.01)
**G01N 33/543** (2006.01)

(21) Application number: **15790812.0**

(22) Date of filing: **24.06.2015**

(86) International application number:
**PCT/US2015/037499**

(87) International publication number:
**WO 2015/200526 (30.12.2015 Gazette 2015/52)**

(54) **PURIFICATION OF NANOPARTICLE-ANTIBODY CONJUGATES**

AUFREINIGUNG VON NANOPARTIKEL-ANTIKÖRPER-KONJUGATEN

PURIFICATION DE CONJUGUÉS NANOPARTICULES-ANTICORPS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.06.2014 US 201462016752 P**

(43) Date of publication of application:
**06.04.2016 Bulletin 2016/14**

(73) Proprietor: **Bio-Rad Laboratories, Inc.**
**Hercules, CA 94547 (US)**

(72) Inventors:
• **BERKELMAN, Tom**
**Hercules, California 94547 (US)**
• **LIAO, Jiali**
**Hercules, California 94547 (US)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**WO-A1-2009/108822      US-A1- 2006 246 524**
**US-A1- 2013 034 854      US-B1- 7 893 177**

US-B2- 7 534 870

• **TERO SOUKKA ET AL: "Supersensitive Time-resolved Immunofluorometric Assay of Free Prostate-specific Antigen with Nanoparticle Label Technology", CLINICAL CHEMISTRY, AMERICAN ASSOCIATION FOR CLINICAL CHEMISTRY, WASHINGTON, DC, vol. 47, no. 7, 1 January 2001 (2001-01-01), pages 1269-1278, XP002294914, ISSN: 0009-9147**
• **SUN, W ET AL.: 'Lyophilization of Semiconducting Polymer Dot Bioconjugates' ANAL CHEM. vol. 85, no. 9, 07 May 2013, ISSN 0003-2700 pages 4316 - 4320, XP055247470**
• **NOUDEH, GD ET AL.: 'Study of the Effects of Polyethylene Glycol Sorbitan Esters Surfactants Group on Biological Membranes' INTERNATIONAL JOURNAL OF PHARMACOLOGY vol. 4, no. 1, 2008, pages 27 - 33, XP055252587**
• **'Sephacryl High Resolution Media HiPrep Sephacryl HR Columns' GE HEALTHCARE 88 AD, [Online] 2008, page 1, XP055247472 Retrieved from the Internet: <URL:https://www.gelifesciences.com/gehcls-images/GELS/ Related%20Content/Files/1326706518989/litdoc18106088_20120116104115.pdf> [retrieved on 2015-08-26]**
• **'Superdex High-Performance Columns' GE HEALTHCARE, [Online] 2006, page 1, XP055247465 Retrieved from the Internet: <URL:http://www.gelifesciences.co.jp/catalog/pdf/18116379.pdf> [retrieved on 2015-09-02]**

## Description

BACKGROUND OF THE INVENTION

P-dot nanoparticles (, e.g., as described in US2012/0282632) are highly fluorescent and are useful reporters when conjugated to antibodies or other proteins. Conjugation reactions between antibodies and nanoparticles are generally carried out using excess antibody in order to assure an optimal conjugation ratio. Following conjugation, the conjugate needs to be separated from the excess free antibody, since unconjugated antibody will compete with the conjugate for target binding. Since free antibodies and nanoparticles are of similar size and density, this presents a challenge. On the other hand, the purification of antibody-nanoparticle conjugates from free antibody using Tween® surfactants in a mixture having an ionic strength of at least 50 nM is known from WO 2009/108820 and T. Soukka et al., Clin. Chem 47(7):1269-78 (2001).

BRIEF SUMMARY OF THE INVENTION

Methods of purifying an antibody-nanoparticle conjugate from free antibody are provided. In a first embodiment, the method comprises providing a mixture of the antibody-nanoparticle conjugate, the free antibody, a poloxamer surfactant, and a buffer, wherein the ionic strength of the mixture is at least 50 mM; contacting the mixture to a polysaccharide-based size exclusion medium to separate the antibody-nanoparticle conjugate from the free antibody; and collecting fractions enriched for the antibody-nanoparticle conjugate from the medium, thereby purifying the antibody-nanoparticle conjugate from the free antibody.

In some embodiments, the polysaccharide-based size exclusion medium comprises agarose.

In some embodiments, the buffer comprises phosphate. In some embodiments, the buffer is phosphate buffered saline (PBS). In some embodiments, the PBS is at a concentration of 0.5-2.0X.

In some embodiments, the antibody is an IgG antibody. In some embodiments, the antibody is a tetrameric IgG antibody.

In some embodiments, the nanoparticle is a polymer dot (p-dot). In some embodiments, the p-dot is 5-100 nm in diameter and is a colloidal semiconducting polymer. In some embodiments, the p-dot is fluorescent.

In some embodiments, the poloxamer surfactant is Pluronic F-68. In some embodiments, the surfactant in the mixture is at a concentration of 0.02%-1.0%.

In some embodiments, the ionic strength of the mixture is 75-300 mM.

In some embodiments, the mixture further comprises free nanoparticle and the medium separates the free nanoparticle from the conjugate.

In a second embodiment, the method comprises providing a mixture of the antibody-nanoparticle conjugate, the free antibody, a poloxamer surfactant, and a buffer, wherein the ionic strength of the mixture is at least 50 mM; contacting the mixture to nanomembrane filter to separate the antibody-nanoparticle conjugate from the free antibody; and collecting fractions enriched for the antibody-nanoparticle conjugate from the filter, thereby purifying the antibody-nanoparticle conjugate from the free antibody.

In some embodiments, the buffer comprises phosphate. In some embodiments, the buffer is phosphate buffered saline (PBS). In some embodiments, the PBS is at a concentration of 0.5-2.0X.

In some embodiments, the antibody is an IgG antibody. In some embodiments, the antibody is a tetrameric IgG antibody.

In some embodiments, the nanoparticle is a polymer dot (p-dot). In some embodiments, the p-dot is 5-100 nm in diameter and is a colloidal semiconducting polymer. In some embodiments, the p-dot is fluorescent.

In some embodiments, the poloxamer surfactant is Pluronic F-68. In some embodiments, the surfactant in the mixture is at a concentration of 0.02%-1.0%.

In some embodiments, the ionic strength of the mixture is 75-300 mM.

In some embodiments, the mixture further comprises free nanoparticle and the medium separates the free nanoparticle from the conjugate.

DEFINITIONS

"Antibody" refers to an immunoglobulin or fragmentary form thereof. The term may include polyclonal or monoclonal antibodies of the classes IgA, IgD, IgE, IgG, and IgM, derived from human or other mammalian cell lines, including natural or genetically modified forms such as humanized, human, single-chain, chimeric, synthetic, recombinant, hybrid, mutated, grafted, and in vitro generated antibodies. "Antibody" may also include composite forms including fusion proteins containing an immunoglobulin moiety. "Antibody" may also include antibody fragments such as Fab, F(ab')2, Fv, scFv, Fd, dAb, Fc and other compositions, whether or not they retain antigen-binding function.

BRIEF DESCRIPTION OF THE DRAWINGS

FIG. 1 is a graph comparing absorbance at 463 nm compared to fraction number. The experiment depicted is purification of a nanoparticle-antibody conjugation sample in low ionic strength 20 mM HEPES-KOH buffer on a 30 cm column of Superose 6.

FIG. 2 is a graph comparing absorbance at 463 nm compared to fraction number. The experiment depicted is purification of a nanoparticle-antibody conjugation sample in IX PBS, 0.1% Pluronic F-68 on a 30 cm column of Superose 6.

DETAILED DESCRIPTION OF THE INVENTION

Antibody-nanoparticle conjugates can be difficult to purify from unconjugated free antibody because the conjugates and free antibodies are of approximately the same size and density, and because nanoparticles can be susceptible to aggregation and precipitation. The inventors have surprisingly found a combination of conditions that allows for separation of antibody-nanoparticle conjugates from free antibody. Namely, the inventors have found that a high ionic strength buffer comprising a poloxamer surfactant can be used to maintain the solubility of the conjugates and to generate sufficient separation of the conjugate from free antibody on polysaccharide-based size exclusion media to allow for purification of the conjugates.

It is believed that the purification method can be applied to a wide range of antibody-nanparticle conjugates. For example, in some embodiments, the antibody is an IgA, IgD, IgE, IgG, and IgM antibody. In some embodiments, the antibody is an antigen-binding antibody fragment such as, for example, a Fab, F(ab')2, or Fv, or a fusion protein comprising such fragments. In some embodiments, the antibody is a single-chain antibody, e.g., a scFv, a fusion of the variable regions of the heavy (VH) and light chains (VL) of one or more antibodies. The antibody can be recombinant or naturally-occurring. The antibody can be human, mouse, rat, rabbit, bovine, goat, camel, or from other antibody-producing species

Nanoparticles are particles in a nanoscale, e.g., from 1 nm to 1000. In some embodiments, the particles are between 1-300 nm, 5-80 nm, or 8-60 nm. Many nanoparticles are roughly spherical in shape, which results in a dimension being the radius or diameter of the spherical particle. The hydrodynamic radius or diameter can also be used to define the nanoparticle size.

In some embodiments, the nanoparticle is a fluorescent semiconducting polymer dot. Examples of such pdots are described in, e.g., Wu, C., et al., Chem. Mater. 21:3816-3822 (2009); Rahim, N. A. A., et al., Adv. Mater. 21:3492-3496 (2009), Rong et al., ACS Nano 7(1):376-84 (2013); patent publications US 2013/0266957; WO 2012/054525; and US 2012/0282632. Chromophoric pdots can be generated by collapsing polymer into a stable submicron sized particle. The nanoparticles provided herein may be formed by any method known in the art for collapsing polymers, including methods relying on precipitation, methods relying on the formation of emulsions (e.g. mini or micro emulsion), and methods relying on condensation.

Nanoparticles can be functionalized as desired to link the nanoparticle to an antibody. Exemplary functionalization of nanoparticles is described in, e.g., US Patent Publication 2012/0282632. As an example, a nanoparticle can be functionalized to present one or more carboxylic acid moieties, which in turn can be used via one or more linker to an antibody. The conjugate components (e.g., antibody and nanoparticle) can be linked covalently or non-covalently. An example of a non-covalent linkage is a biotin-streptavidin affinity, where one member of the conjugate is biotinylated and the other member of the conjugate is linked to streptavidin. Other examples of linkage options include direct coupling of nanoparticles to antibody amines; modification of nanoparticles with maleimide and subsequent linkage to an antibody having an exposed thiol (generated, for example, by treating the antibody with mercaptoethylamine or 2-iminothiolane (Traut's reagent)); modification of nanoparticles with hydrazine and linkage to an antibody with oxidized glycan (aldehyde); or use of click chemistry (e.g., modification of nanoparticles with strained alkyne and linkage to an antibody modified with azide).

Any type of conjugation methods can be used for conjugating an antibody to a nanoparticle. Generally, to generate a desired yield of conjugate, an excess of antibody is provided in the conjugation reaction. This can result in a significant amount of free (unconjugated) antibody following the conjugation reaction. In some embodiments, there is also an amount of free unconjugated nanoparticles in the reaction mixture. The methods described herein are useful to purifying the conjugates from the free unconjugated members of the conjugation reaction. In some embodiments, a reagent is applied that will react with remaining reactive groups and prevent further reaction. As an example, conjugation between a maleimide-functionalized nanonparticle and a thiolated or reduced antibody will be stopped or quenched with an alkylating reagent including N-ethylmaleimide. Reaction between an NHS-appended nanoparticle and a protein will be stopped or quenched with an amine including ethanolamine

Once a conjugation has been performed, the resulting conjugation mixture (nanoparticle/antibody conjugate and unreacted free antibody and optionally free nanoparticle) is adjusted to have an ionic strength of at least 50 mM (e.g., between 50-500mM, 50-300 mM, 100-300 mM). For example, the mixture's ionic strength can be adjusted with a high ionic strength buffer, e.g., a buffer containing a high concentration of ions to maintain the ionic strength listed above. In some embodiments, the buffer comprises at least 50 or 100 mM $Na^+$ or $K^+$. In some embodiments the buffer comprises phosphate ($PO_4^{3-}$). An exemplary buffer is phosphate buffered saline (PBS) (1X PBS = 10 mM sodium phosphate, 150 mM sodium chloride pH 7.8). In some embodiments, 0.5-3X, e.g., 0.5-2.0X, e.g., 1X PBS is included in the mixture.

Ionic strength is calculated according to the following formula:

$$I = \frac{1}{2}\sum_{i=1}^{n} c_i z_i^2 \; ,$$

where $c_i$ is the molar concentration of ion i (M, mol/1), $z_i$ is the charge number of that ion, and the sum is taken over all ions in the solution.

Also included in the mixture is a sufficient amount of a poloxamer surfactant to prevent aggregation and precipitation of the conjugates from the mixture, especially upon introduction of the high ionic strength buffer, which might otherwise result in aggregation or precipitation of the conjugates. Poloxamer surfactants are characterized by a central hydrophobic chain of polyoxypropylene (poly(propylene oxide)) flanked by two hydrophilic chains of polyoxyethylene (poly(ethylene oxide)). Because the lengths of the polymer blocks can be customized, many different poloxamers exist that have slightly different properties. Poloxamer copolymers are commonly named with the letter "P" (for poloxamer) followed by three digits, the first two digits x 100 give the approximate molecular mass of the polyoxypropylene core, and the last digit x 10 gives the percentage polyoxyethylene content (e.g., P407 = Poloxamer with a polyoxypropylene molecular mass of 4,000 g/mol and a 70% polyoxyethylene content). For the Pluronic and Synperonic poloxamer tradenames, coding of these copolymers starts with a letter to define its physical form at room temperature (L = liquid, P = paste, F = flake (solid)) followed by two or three digits. The first digit (two digits in a three-digit number) in the numerical designation, multiplied by 300, indicates the approximate molecular weight of the hydrophobic chain; and the last digit x 10 gives the percentage polyoxyethylene content (e.g., F-68 indicates a polyoxypropylene molecular mass of 1,800 g/mol and a 80% polyoxyethylene content). Exemplary poloxamer surfactants include, Pluronics F-68. The concentration of the surfactant used can be determined empirically (i.e., titrated such that precipitation of the conjugates does not occur). In some embodiments, the concentration of surfactant is 0.02%-1%, e.g., 0.05-0.2%, e.g., 0.1%.

The buffered high ionic strength mixture comprising the poloxamer surfactant, conjugates, and free antibody are subsequently applied to a polysaccharide-based (i.e., comprising polysaccharides) size exclusion medium to separate the conjugate from free antibody and optionally from free nanoparticle. Size exclusion media when used in chromatography separates molecules based on their size or molecular weight. Size exclusion chromatography is based on the selective permeation of soluble proteins or other target molecules through a column of particles of a particular size, which particles have pores, typically, but not always, of a known size. Proteins of a size larger than the pores will not enter the pores. Large proteins that do not enter the pores pass around the particles and are eluted in the void volume (Vo). Very small proteins and salts are retained within the particles until the total permeation volume (Vt) is reached. Proteins that elute between the void volume and the total permeation volume are resolved, based upon the size and shape of their molecules.

In some embodiments, the polysaccharide in the polysaccharide-based size exclusion medium is agarose. In some embodiments, the polysaccharide is dextran. An exemplary agarose-based size exclusion medium is Superose 6 or 12 (available from GE Healthcare). In some cases, the medium will be provided in a column with the sample applied to the top of the chromatography and gravity forcing the sample through the column. In other embodiments, artificial pressure (e.g., HPLC) can also be applied as desired.

The output from the gel exclusion medium can be monitored for the presence of the conjugate, free antibody, or other components of the sample as desired to determine fractions that contain the conjugate and that are free, or at least have a reduced amount, of free antibody compared to the original conjugation mixture. In some embodiments, at least 90%, 95%, 99% of the untreated antibody in the conjugation reaction is removed in the resulting purified conjugate fractions. Exemplary methods for measuring output include monitoring a characteristic absorbance wavelength for the nanoparticle or antibody. The term "fraction" is used to refer to a portion of the output of chromatography and is not intended to limit how the output is collected or whether the output is collected in parts or continuously.

As an alternative to size-exclusion media, the high ionic strength, surfactant-containing conjugation mixture can be applied to a nanomembrane filter to separate the conjugates from free antibody. In these embodiments, the nanopores of the nanomembrane are selected to prevent passage of the conjugates while allowing for passage of the unconjugated antibody. The conjugation reaction can be passed through a nanoporous membrane such that conjugated nanoparticles will be retained and unconjugated antibodies will pass through the membrane. Repeated dilution and re-filtration will result in a preparation that is substantially free of unreacted antibody (e.g., at least 90%, 95%, 99% of the untreated antibody in the conjugation reaction is removed). The size of the nanopores will depend upon the size of the nanoparticle in the conjugate.

EXAMPLE

An initial attempt to purify an antibody-nanopore conjugate from conjugation reactants such as free antibody was performed. High-resolution size exclusion chromatography on media with an appropriately high exclusion limit was attempted, however it was found that P-dot nanoparticles bound to many commercial media and would not elute from the columns. It was found that this binding was not significant on Superose 6 (GE Healthcare), Sephacryl 400, and Superdex 200.

Initial attempts to purify the conjugate on Sephacryl 400 and Superdex 200, however, were not successful. In these attempts, a nanoparticle-antibody conjugation sample in a low ionic strength 20 mM HEPES-KOH buffer was applied to 10 cm columns of Sephacryl 400 or Superdex 200. Separation of the conjugates from free antibody was poor (data not shown). P-dot was monitored by absorbance at 463 nm. IgG was monitored by densitometry on native gel.

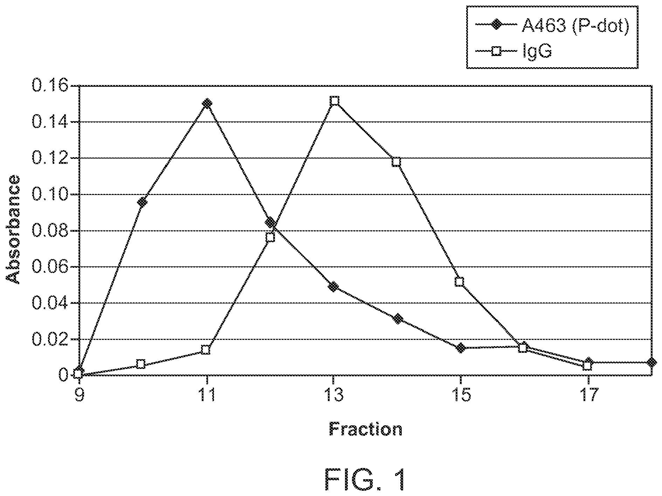

A 10 cm gravity column of Superose 6, which is an agarose-based size exclusion medium, was prepared. A nanoparticle-antibody conjugation sample in low ionic strength 20 mM HEPES-KOH buffer was added to the column, but did not result in helpful separation. The same experiment was repeated, but with a 30 cm column of Superose 6. While the peaks of the conjugate was separated from the free antibody, there was significant overlap of the shoulders of the peaks (see FIG. 1) and thus the conditions did not allow for optimal purification of the conjugate.

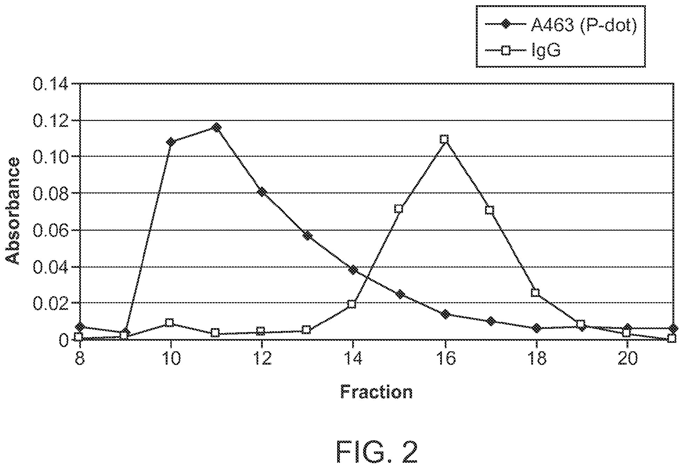

A higher ionic strength buffer (1X PBS) was tested with the conjugate, but the higher ionic strength of the buffer resulted in precipitation of the conjugate, thereby preventing purification. A further mixture was prepared in IX PBS, but also including 0.1% Pluronic F-68. The conjugate remained in solution in this mixture and was applied to a 30 cm Superose 6 column. The resulting separation of the conjugate and free antibody (FIG. 2) was significantly better than the separation observed for the HEPES-KOH buffer mixture and allowed for purification of the antibody conjugates from free antibody.

**Claims**

1. A method of purifying an antibody-nanoparticle conjugate from free antibody, the method comprising, providing a mixture of the antibody-nanoparticle conjugate, the free antibody, a poloxamer surfactant, and a buffer, wherein the ionic strength of the mixture is at least 50 mM; contacting the mixture to a polysaccharide-based size exclusion medium to separate the antibody-nanoparticle conjugate from the free antibody; and collecting fractions enriched for the antibody-nanoparticle conjugate from the medium, thereby purifying the antibody-nanoparticle conjugate from the free antibody.

2. The method of claim 1, wherein

   (i) the polysaccharide-based size exclusion medium comprises agarose; or
   (ii) the ionic strength of the mixture is 75-300 mM.

3. The method of claim 1, wherein

   (i) the buffer comprises phosphate; or
   (ii) the buffer is phosphate buffered saline (PBS), preferably the PBS is at a concentration of 0.5-2.0X.

4. The method of claim 1, wherein

   (i) the antibody is an IgG antibody; or
   (ii) the antibody is a tetrameric IgG antibody.

5. The method of claim 1, wherein the nanoparticle is a polymer dot (p-dot), preferably

   (i) the p-dot is 5-100 nm in diameter and is a colloidal semiconducting polymer; and/or
   (ii) the p-dot is fluorescent.

6. The method of claim 1, wherein the poloxamer surfactant is Pluronic F-68.

7. The method of claim 1 or 6, wherein the poloxamer surfactant in the mixture is at a concentration of 0.02%-1.0%.

8. The method of any of the foregoing claims, wherein the mixture further comprises free nanoparticle and the medium separates the free nanoparticle from the conjugate.

9. A method of purifying an antibody-nanoparticle conjugate from free antibody, the method comprising, providing a mixture of the antibody-nanoparticle conjugate, the free antibody, a poloxamer surfactant, and a buffer, wherein the ionic strength of the mixture is at least 50 mM; contacting the mixture to nanomembrane filter to separate the antibody-nanoparticle conjugate from the free antibody; and collecting fractions enriched for the antibody-nanoparticle conjugate from the filter, thereby purifying the antibody-nanoparticle conjugate from the free antibody.

10. The method of claim 9, wherein

    (i) the buffer comprises phosphate; or
    (ii) the buffer is phosphate buffered saline (PBS), preferably the PBS is at a concentration of 0.5-2.0X; or
    (iii) the ionic strength of the mixture is 75-300 mM.

11. The method of claim 9, wherein

    (i) the antibody is an IgG antibody; or
    (ii) the antibody is a tetrameric IgG antibody.

12. The method of claim 9, wherein the nanoparticle is a polymer dot (p-dot), preferably

> (i) the p-dot is 5-100 nm in diameter and is a colloidal semiconducting polymer; and/or
> (ii) the p-dot is fluorescent.

13. The method of claim 9, wherein the poloxamer surfactant is Pluronic F-68.

14. The method of claim 9 or 13, wherein the poloxamer surfactant in the mixture is at a concentration of 0.02%-1.0%.

15. The method of any of claims 9-14, wherein the mixture further comprises free nanoparticle and the medium separates the free nanoparticle from the conjugate.


**Patentansprüche**

1. Verfahren zum Reinigen eines Antikörper-Nanopartikel-Konjugats von freiem Antikörper, wobei das Verfahren umfasst:

> Bereitstellen eines Gemischs des Antikörper-Nanopartikel-Konjugats, des freien Antikörpers, eines Poloxamer-Tensids und eines Puffers, wobei die Ionenstärke des Gemischs wenigstens 50 mM beträgt;
> In-Kontakt-Bringen des Gemischs mit einem Größenausschlussmedium auf Polysaccharidbasis, um das Antikörper-Nanopartikel-Konjugat von dem freien Antikörper zu trennen; und
> Gewinnen von mit dem Antikörper-Nanopartikel-Konjugat angereicherten Fraktionen aus dem Medium, wodurch das Antikörper-Nanopartikel-Konjugat von dem freien Antikörper gereinigt wird.

2. Verfahren gemäß Anspruch 1, wobei

> (i) das Größenausschlussmedium auf Polysaccharidbasis Agarose umfasst; oder
> (ii) die Ionenstärke des Gemischs 75-300 mM beträgt.

3. Verfahren gemäß Anspruch 1, wobei

> (i) der Puffer Phosphat umfasst; oder
> (ii) es sich bei dem Puffer um phosphatgepufferte Kochsalzlösung (PBS) handelt, wobei vorzugsweise die PBS in einer Konzentration von 0,5-2,0 x vorliegt.

4. Verfahren gemäß Anspruch 1, wobei

> (i) der Antikörper ein IgG-Antikörper ist; oder

> (ii) der Antikörper ein tetramerer IgG-Antikörper ist.

5. Verfahren gemäß Anspruch 1, wobei der Nanopartikel ein Polymer-Dot (p-Dot) ist, wobei vorzugsweise

> (i) der p-Dot einen Durchmesser von 5-100 nm aufweist und ein kolloidales halbleitendes Polymer ist; und/oder
> (ii) der p-Dot fluoreszierend ist.

6. Verfahren gemäß Anspruch 1, wobei es sich bei dem Poloxamer-Tensid um Pluronic F-68 handelt.

7. Verfahren gemäß Anspruch 1 oder 6, wobei das Poloxamer-Tensid in dem Gemisch in einer Konzentration von 0,02% bis 1,0% vorliegt.

8. Verfahren gemäß einem der vorstehenden Ansprüche, wobei das Gemisch weiterhin freie Nanopartikel umfasst und das Medium die freien Nanopartikel von dem Konjugat trennt.

9. Verfahren zum Reinigen eines Antikörper-Nanopartikel-Konjugats von freiem Antikörper, wobei das Verfahren umfasst:

> Bereitstellen eines Gemischs des Antikörper-Nanopartikel-Konjugats, des freien Antikörpers, eines Poloxamer-Tensids und eines Puffers, wobei die Ionenstärke des Gemischs wenigstens 50 mM beträgt;
> In-Kontakt-Bringen des Gemischs mit einem Nanomembranfilter, um das Antikörper-Nanopartikel-Konjugat von dem freien Antikörper zu trennen; und
> Gewinnen von mit dem Antikörper-Nanopartikel-Konjugat angereicherten Fraktionen aus dem Filter, wodurch das Antikörper-Nanopartikel-Konjugat von dem freien Antikörper gereinigt wird.

10. Verfahren gemäß Anspruch 9, wobei

> (i) der Puffer Phosphat umfasst; oder
> (ii) es sich bei dem Puffer um phosphatgepufferte Kochsalzlösung (PBS) handelt, wobei vorzugsweise die PBS in einer Konzentration von 0,5-2,0 x vorliegt; oder
> (iii) die Ionenstärke des Gemischs 75-300 mM beträgt.

11. Verfahren gemäß Anspruch 9, wobei

> (i) der Antikörper ein IgG-Antikörper ist; oder
> (ii) der Antikörper ein tetramerer IgG-Antikörper ist.

**12.** Verfahren gemäß Anspruch 9, wobei der Nanopartikel ein Polymer-Dot (p-Dot) ist, wobei vorzugsweise

   (i) der p-Dot einen Durchmesser von 5-100 nm aufweist und ein kolloidales halbleitendes Polymer ist; und/oder
   (ii) der p-Dot fluoreszierend ist.

**13.** Verfahren gemäß Anspruch 9, wobei es sich bei dem Poloxamer-Tensid um Pluronic F-68 handelt.

**14.** Verfahren gemäß Anspruch 9 oder 13, wobei das Poloxamer-Tensid in dem Gemisch in einer Konzentration von 0,02% bis 1,0% vorliegt.

**15.** Verfahren gemäß einem der Ansprüche 9 bis 14, wobei das Gemisch weiterhin freie Nanopartikel umfasst und das Medium die freien Nanopartikel von dem Konjugat trennt.


**Revendications**

**1.** Procédé de purification d'un conjugué anticorps-nanoparticule de l'anticorps libre, le procédé comprenant :

   l'obtention d'un mélange du conjugué anticorps-nanoparticule, de l'anticorps libre, d'un tensioactif poloxamère et d'un tampon, dans lequel la force ionique du mélange est d'au moins 50 mM ;
   la mise en contact du mélange avec un milieu d'exclusion stérique à base de polysaccharide pour séparer le conjugué anticorps-nanoparticule de l'anticorps libre ; et
   la collecte des fractions enrichies en conjugué anticorps-nanoparticule à partir du milieu, purifiant ainsi le conjugué anticorps-nanoparticule de l'anticorps libre.

**2.** Procédé selon la revendication 1, dans lequel

   (i) le milieu d'exclusion stérique à base de polysaccharide comprend de l'agarose ; ou
   (ii) la force ionique du mélange est de 75-300 mM.

**3.** Procédé selon la revendication 1, dans lequel

   (i) le tampon comprend du phosphate ; ou
   (ii) le tampon est une solution salée tamponnée au phosphate (STP), de préférence, la STP est à une concentration de 0,5-2,0X.

**4.** Procédé selon la revendication 1, dans lequel

   (i) l'anticorps est un anticorps IgG ; ou
   (ii) l'anticorps est un anticorps IgG tétramérique.

**5.** Procédé selon la revendication 1, dans lequel la nanoparticule est un point polymère (« polymer dot » ou « p-dot »), de préférence

   (i) le p-dot a un diamètre de 5-100 nm et est un polymère semi-conducteur colloïdal ; et/ou
   (ii) le p-dot est fluorescent.

**6.** Procédé selon la revendication 1, dans lequel le tensioactif poloxamère est le Pluronic F-68.

**7.** Procédé selon la revendication 1 ou 6, dans lequel le tensioactif poloxamère est à une concentration de 0,02%-1,0% dans le mélange.

**8.** Procédé selon l'une quelconque des revendications précédentes, dans lequel le mélange comprend en outre de la nanoparticule libre et le milieu sépare la nanoparticule libre du conjugué.

**9.** Procédé de purification d'un conjugué anticorps-nanoparticule de l'anticorps libre, le procédé comprenant :

   l'obtention d'un mélange du conjugué anticorps-nanoparticule, de l'anticorps libre, d'un tensioactif poloxamère et d'un tampon, dans lequel la force ionique du mélange est d'au moins 50 mM ;
   la mise en contact du mélange avec un filtre à nanomembrane pour séparer le conjugué anticorps-nanoparticule de l'anticorps libre ; et
   la collecte des fractions enrichies en conjugué anticorps-nanoparticule à partir du filtre, purifiant ainsi le conjugué anticorps-nanoparticule de l'anticorps libre.

**10.** Procédé selon la revendication 9, dans lequel

   (i) le tampon comprend du phosphate ; ou
   (ii) le tampon est une solution salée tamponnée au phosphate (STP), de préférence, la STP est à une concentration de 0,5-2,0X ; ou
   (iii) la force ionique du mélange est de 75-300 mM.

**11.** Procédé selon la revendication 9, dans lequel

   (i) l'anticorps est un anticorps IgG ; ou
   (ii) l'anticorps est un anticorps IgG tétramérique.

**12.** Procédé selon la revendication 9, dans lequel la nanoparticule est un point polymère (« polymer dot » ou « p-dot »), de préférence

(i) le p-dot a un diamètre de 5-100 nm et est un polymère semi-conducteur colloïdal ; et/ou
(ii) le p-dot est fluorescent.

13. Procédé selon la revendication 9, dans lequel le tensioactif poloxamère est le Pluronic F-68.

14. Procédé selon la revendication 9 ou 13, dans lequel le tensioactif poloxamère est à une concentration de 0,02%-1,0% dans le mélange.

15. Procédé selon l'une quelconque des revendications 9-14, dans lequel le mélange comprend en outre de la nanoparticule libre et le milieu sépare la nanoparticule libre du conjugué.

FIG. 1

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120282632 A
- WO 2009108820 A
- US 20130266957 A
- WO 2012054525 A

**Non-patent literature cited in the description**

- **T. SOUKKA et al.** *Clin. Chem,* 2001, vol. 47 (7), 1269-78
- **WU, C. et al.** *Chem. Mater.,* 2009, vol. 21, 3816-3822
- **RAHIM, N. A. A. et al.** *Adv. Mater.,* 2009, vol. 21, 3492-3496
- **RONG et al.** *ACS Nano,* 2013, vol. 7 (1), 376-84